(19) 

Europäisches
Patentamt
European
Patent Office
Office européen
des brevets

(11) **EP 1 843 269 A1**

(12) **EUROPEAN PATENT APPLICATION**

(43) Date of publication:
**10.10.2007 Bulletin 2007/41**

(51) Int Cl.:
***G06F 19/00*** *(2006.01)*

(21) Application number: **07005630.4**

(22) Date of filing: **19.03.2007**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK YU**

(30) Priority: **06.04.2006 JP 2006104802**

(71) Applicant: **Hitachi, Ltd.
Chiyoda-ku
Tokyo 100-8280 (JP)**

(72) Inventors:
• **Saito, Akira
Tokyo 100-8220 (JP)**
• **Tanikawa, Koji
Tokyo 100-8220 (JP)**
• **Kikkawa, Emiko
Tokyo 100-8220 (JP)**

(74) Representative: **Strehl Schübel-Hopf & Partner
Maximilianstrasse 54
80538 München (DE)**

(54) **Pharmacokinetic analysis system and method thereof**

(57) Systems and methods for analyzing pharma-cokinetics in such a manner that the influence of genetic polymorphisms of an individual are taken into consideration, and for allowing implementation of high-accuracy haplotype frequency estimation and diplotype configuration estimation even in cases where the number of individuals is small from which data is obtainable when making pharmacokinetic analyses.

FIG. 3

**Description**

BACKGROUND OF THE INVENTION

Field of the Invention

The present invention relates to a pharmacokinetic analysis system and a method of the pharmacokinetic analysis for analyzing transition of in-blood drug concentration of an individual in such a manner that the influence of genetic polymorphism is taken into consideration.

Description of the Related Art

A drug dosed to humans passes through processes of liberation, absorption, distribution, metabolism, and excretion. The research field regarding transition of drug concentration in body fluid (mainly in blood) in these processes is referred to as "Pharmacokinetics: PK)". When investigating pharmacokinetics in humans, data on the pharmacokinetics, such as in-blood drug concentration or in-urine excretion amount obtained from a clinical trial, is analyzed using statistics. After that, the pharmacokinetics is investigated based on this analysis result.
When applying the pharmacokinetics analysis to the data on the in-blood drug concentration obtained from a clinical trial, the following two cases are conceivable: A case of estimating a pharmacokinetic parameter for each examinee (individual), and a case of estimating a pharmacokinetic parameter in a population out of which an individual is extracted. In the former case, a method is generally used where the measurements of the in-blood drug concentration are made for each individual at large number of points-in-time, and where the pharmacokinetic parameter is estimated for each individual on the basis of the data obtained. An early-stage clinical trial, such as clinical phase-Itrial, is a typical example of this former case. The latter case is an analysis method commonly referred to as "population pharmacokinetic analysis". In the population pharmacokinetic analysis, the pharmacokinetics in a population out of which each individual is extracted is analyzed based on the data on the individual.
In general, the data on the pharmacokinetics exhibits large variations between individuals and inside an individual. Even if a constant amount of drug is dosed, the in-blood drug concentrations differ inside an individual or between individuals. Also, even if the dose is adjusted so that the in-blood drug concentrations will become the same, the effect and action of the drug will not necessarily become the same. In this way, as one of the causes by which the in-blood drug concentrations and the effect and action of the drug differ between individuals, it is conceivable that workings of genes related with the various processes described above differ depending on the individuals. As one of accomplishments of the Human Genome Project, it is conceivable that individual genetic information (i.e., the individual genetic polymorphism) is utilized in the field of Pharmacokinetics. Expectations are now getting more and more placed on this utilization of the individual genetic information as an effective method for making it possible to understand the problem of an individual difference in a drug therapy more scientifically than ever.
Statistical genetics method is one of the effective methods for analyzing the relationship between the genetic polymorphism of an individual and occurrence of diseases and side effects of drugs. The statistical genetics method is a genetic name for methodologies of searching for the genetic polymorphism related with a specific trait (presence or absence of a disease, and presence or absence of side effect of a drug) by taking advantage of statistics. The statistical genetics method is the methodologies onto which attention has been focused in recent years.
The statistical genetics method is classified into a linkage analysis method and an analysis method using linkage disequilibrium. The former is the analysis method using the linkage, i.e., an exception of Mendel's Independent Law. The latter is the analysis method using the linkage disequilibrium, i.e., a non-independent phenomenon of a plurality of genetic polymorphisms within a group. Family-line information is needed in the linkage analysis method; whereas the family-line information is not necessarily needed in the analysis method using the linkage disequilibrium. On account of this, in recent years, the analysis method using the linkage disequilibrium has become prevalent. In the analysis method using the linkage disequilibrium, frequencies of a genetic polymorphism on which attention is focused, or frequencies of a combination (haplotype) of a plurality of genetic polymorphisms in an area where the linkage disequilibrium exists are compared between groups of different traits (case group and control group, and responder group to a drug and non-responder group thereto). This comparison allows investigation of the relationship between a specific trait and the genetic polymorphisms or haplotype.
In the analysis method using the linkage disequilibrium, in general, the analysis is made based on the haplotype or diplotype configuration (a combination of two haplotypes which an individual has). Up to the present, several proposals have been made concerning methodologies for estimating the haplotype frequency of a population and the diplotype configuration of an individual on the basis of the data on the genetic polymorphism of the individual. The representative proposals are a methodology using EM algorithm as described in Excoffier L & Slatkin M: Maximum-likelihood estimation of molecular haplotype frequencies in a diploid population, Mol Biol Evol, Vol. 12, pp. 921-927, 1995, and PHASE method as described in Stephens M et al.: A new statistical method for haplotype reconstruction from population data, Am J Hum Genet, Vol. 68, pp. 978-989, 2001.

As described above, it is conceivable that the genetic polymorphism of an individual is utilized in the field of Pharmacokinetics. Expectations are now getting more and more placed on this utilization of the genetic polymorphism as the effective method for making it possible to understand the problem of an individual difference in a drug therapy more scientifically than ever. However, a method for analyzing the pharmacokinetics in such a manner that the influence of the genetic polymorphism of an individual is taken into consideration has been not established yet.

Also, the estimation methodologies reported up to the present for estimating the haplotype frequency of a population and the diplotype configuration of an individual require the genetic polymorphism data on a large number of individuals (a few hundreds to a few thousands of individuals in general) in order to enhance the estimation accuracy. Usually, however, the number of individuals obtainable in a clinical trial or the like is about a few tens of individuals. On account of this, acquiring the genetic polymorphism data on a large number of individuals is difficult in the pharmacokinetic analysis. Accordingly, there has existed a problem that the estimation accuracy is insufficient in estimating the haplotype frequency of a population and the diplotype configuration of an individual.

SUMMARY OF THE INVENTION

In view of this situation, an object of the present invention is to solve the above-described problems, and to provide the method for analyzing the pharmacokinetics in such a manner that the influence of the genetic polymorphism of an individual is taken into consideration. Also, another object of the present invention is to provide a method for allowing implementation of high-accuracy haplotype frequency estimation and diplotype configuration estimation even in the case where the number of individuals is small from which the data is obtainable when making the pharmacokinetic analysis.

The pharmacokinetic analysis system and method of the present invention estimate the diplotype configuration of an individual, and expresses a pharmacokinetic parameter as a function of the diplotype configuration of the individual. This processing makes it possible to configure pharmacokinetic models, and to make the pharmacokinetic analysis where the influence of the genetic polymorphism of the individual is taken into consideration. Also, the system and method of the present invention create the individual in a pseudo manner on the basis of information on the haplotype frequency and the number of individuals obtainable from documents, empirical knowledge, and the like. Then, using the data created, the system and method correct the estimation result of the diplotype configuration of the individual, thereby implementing an enhancement in the estimation accuracy. Moreover, the system and method select an optimum model from among the pharmacokinetic models, and displays a drug-concentration time course curve based on the selected model in a manner of being equipped with a confidence interval. Then, the system and method permit a user to set the confidence interval freely. This processing makes it possible to perform stratification of patients depending on a purpose.

According to the pharmacokinetic analysis method of the present invention, it becomes possible to analyze the pharmacokinetics in such a manner that the influence of the genetic polymorphism of an individual is taken into consideration. Also, it becomes possible to investigate in detail the problem of an individual difference in a drug therapy.

Other objects, features and advantages of the invention will become apparent from the following description of the embodiments of the invention taken in conjunction with the accompanying drawings.

BRIEF DESCRIPTION OF THE DRAWINGS

Fig. 1 is a diagram for illustrating an example of the device configuration of a pharmacokinetic analysis device;

Fig. 2 is a diagram for illustrating an example of the embodiment of a pharmacokinetic analysis program;

Fig. 3 is a diagram for illustrating an example of the embodiment of the pharmacokinetic analysis program in a case of including a step of correcting the estimation result of the diplotype configuration;

Fig. 4 is a diagram for illustrating an example of the screen of the pharmacokinetic analysis program;

Fig. 5 is a diagram for illustrating an example of the setting screen of a population pharmacokinetic model building condition and a stratification condition;

Fig. 6 is a diagram for illustrating an example of the setting screen of the haplotype frequency, sample size, and weighting coefficient;

Fig. 7 is a diagram for illustrating an example of the data structure of genetic data;

Fig. 8 is a diagram for illustrating an example of the data structure of the pharmacokinetic data and clinical data;

Fig. 9 is a diagram for illustrating an example of the embodiment of a diplotype configuration estimation step;

Fig. 10 is a diagram for illustrating an example of the embodiment of the diplotype configuration correction step;

Fig. 11 is a diagram for illustrating effectiveness of the diplotype-configuration estimation result correction made by the diplotype configuration correction step;

Fig. 12 is a diagram for illustrating an example of the embodiment of the pharmacokinetic model building step and the pharmacokinetic parameter estimation step;

Fig. 13 is a diagram for illustrating a description example of a diagnosis-assisting knowledge database 15;

Fig. 14 is a diagram for illustrating a 1-compartment model of oral administration;

Fig. 15 is a diagram for illustrating an example of the device configuration of the pharmacokinetic analysis device in the case of including the step of correcting the estimation result of the diplotype configuration;

Fig. 16 is a diagram for illustrating an example of the screen for selecting an optimum pharmacokinetic model; and

Fig. 17 is a diagram for illustrating an example of the display screen of a stratification-condition satisfaction judgment result.

## DESCRIPTION OF THE INVENTION

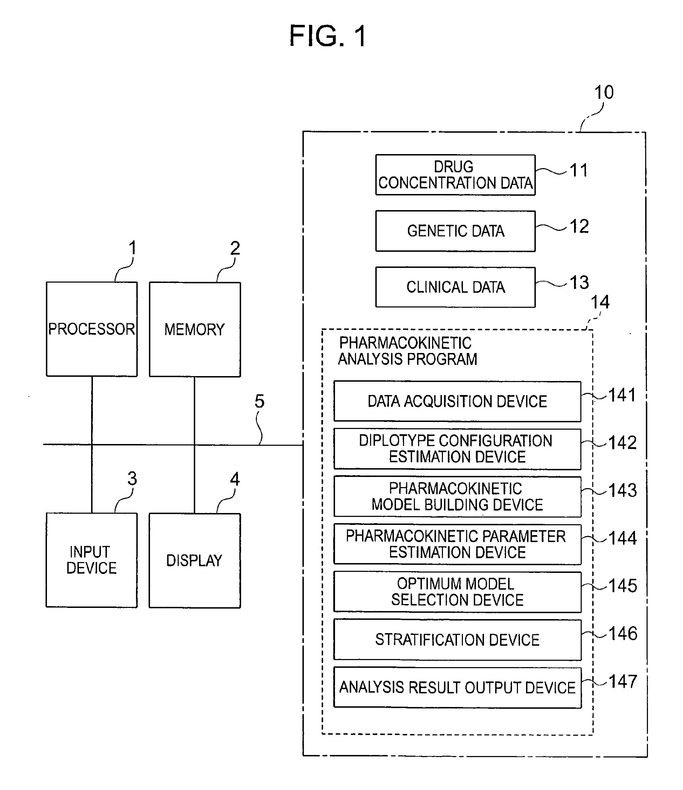

Fig. 1 is a diagram for illustrating an example of the device configuration of a pharmacokinetic analysis device according to the present invention. The pharmacokinetic analysis device according to the present invention is mainly configured by an electronic computer such as the so-called personal computer. A processor 1, a memory 2, an input device 3, a display 4, and an external storage device 10 are connected to a system bus 5. The external storage device 10 includes therein drug concentration data 11 on a plurality of individuals obtained from a clinical trial or the like, genetic data 12 on the plurality of individuals, clinical data 13 on the plurality of individuals, and a pharmacokinetic analysis program 14 for making the pharmacokinetic analysis according to the present invention. In addition to these, the device 10 of course includes a database and processing programs needed for implementing functions as the electronic computer.

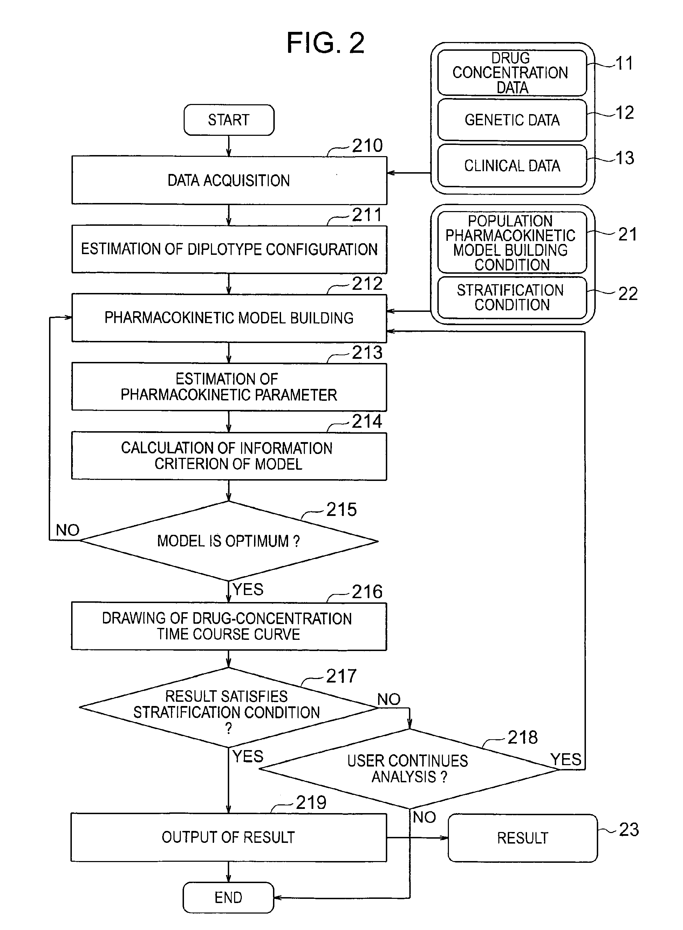

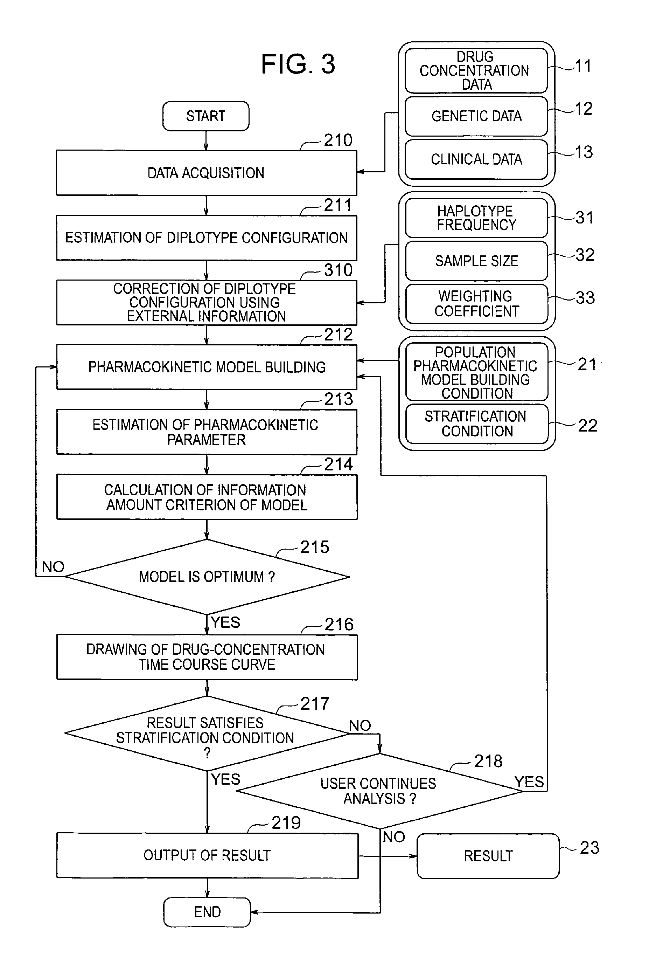

The pharmacokinetic analysis program 14 includes a data acquisition device 141 for acquiring the drug concentration data 11, the genetic data 12, and the clinical data 13 on a plurality of individuals, a diplotype configuration estimation device 142 for estimating diplotype configurations of the individuals on the basis of the genetic data 12 on the plurality of individuals acquired by the data acquisition device 141, a pharmacokinetic model building device 143 for building pharmacokinetic models on the basis of the diplotype configurations of the individuals estimated by the diplotype configuration estimation device 142, a pharmacokinetic parameter estimation device 144 for estimating a pharmacokinetic parameter on the basis of the data acquired by the data acquisition device 141 and the pharmacokinetic models built by the pharmacokinetic model building device 143, an optimum model selection device 145 for calculating information criterion of the pharmacokinetic models on the basis of the pharmacokinetic models built by the pharmacokinetic model building device 143 and the pharmacokinetic parameter estimated by the pharmacokinetic parameter estimation device 144, judging whether or not the pharmacokinetic models built by the pharmacokinetic model building device 143 are optimum on the basis of the information criterion calculated, and selecting an optimum pharmacokinetic model, a stratification device 146 for creating a drug-concentration time course curve on the basis of the optimum pharmacokinetic model selected by the optimum model selection device 145, and performing stratification of the drug-concentration time course curve, and an analysis result output device 147 for outputting, as an analysis result, the data on the drug-concentration time course curve created by the stratification device 146. Although the program like this has been described assuming that the program exists in the external storage device 10, the program may also be installed into the present device via a medium. Hereinafter, referring to Fig. 2 and Fig. 3, the explanation will be given below concerning the embodiment of the pharmacokinetic analysis program 14.

Fig. 2 is a diagram for illustrating an example of the embodiment of the pharmacokinetic analysis based on the pharmacokinetic analysis program 14. Hereinafter, the explanation will be given below concerning each step in Fig. 2.

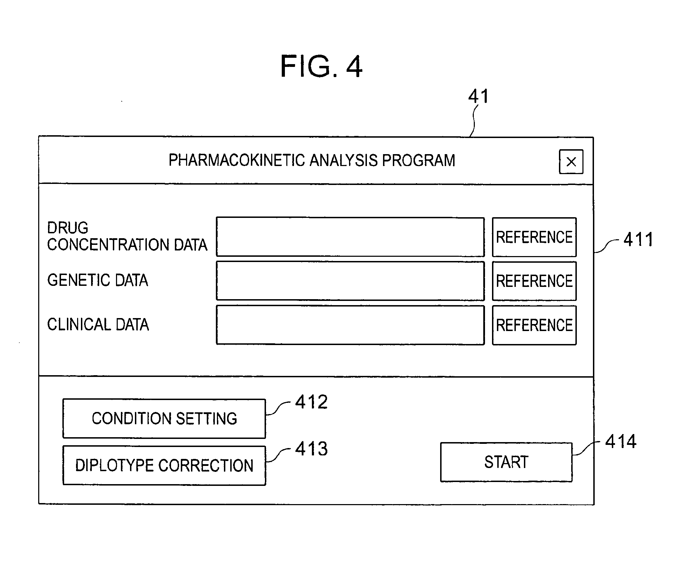

A data acquisition step 210: The data acquisition device 141 acquires the drug concentration data 11, the genetic data 12, and the clinical data 13 on a plurality of individuals. The data to be acquired are specified by the user via a setting screen which will be explained later using Fig. 4.

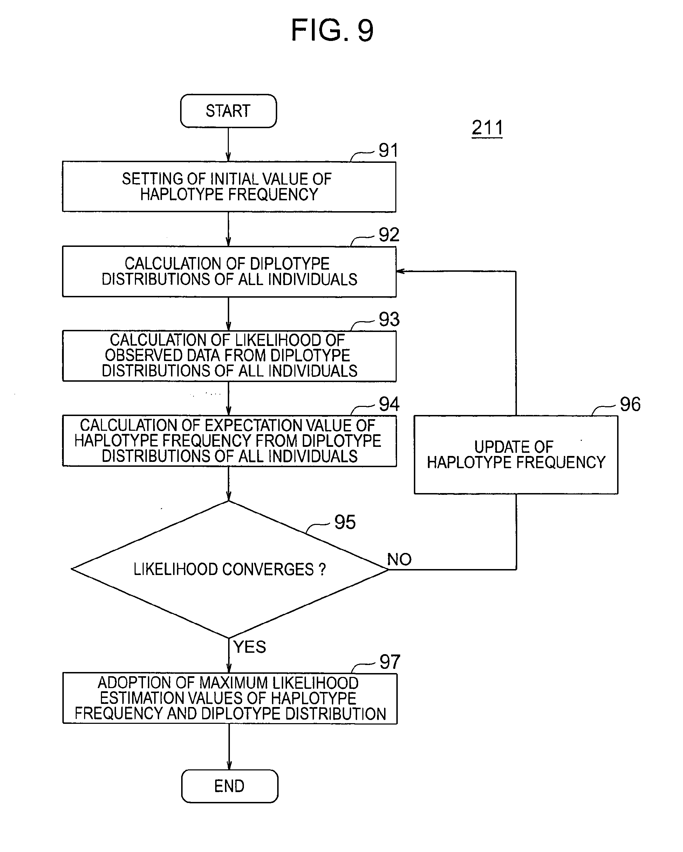

A diplotype configuration estimation step 211: The diplotype configuration estimation device 142 estimates diplotype configurations of the individuals on the basis of the genetic data 12 on the plurality of individuals acquired at the data acquisition step 210. The details of the diplotype configuration estimation step 211 will be explained later using Fig. 9.

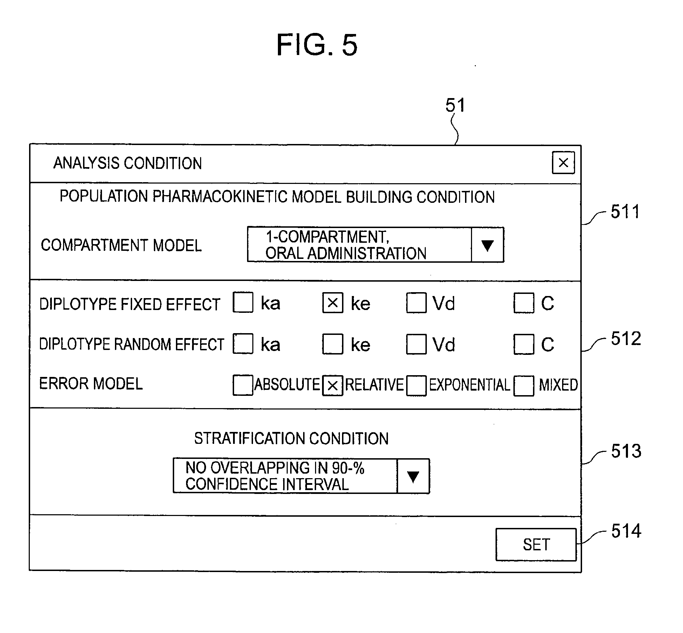

A pharmacokinetic model building step 212: The pharmacokinetic model building device 143 builds pharmacokinetic models on the basis of a population pharmacokinetic model building condition 21 and a stratification condition 22, i.e., the condition for performing stratification of patients. The population pharmacokinetic model building condition 21 and the stratification condition 22 are specified by the user via a setting screen which will be explained later using Fig. 5.

A pharmacokinetic parameter estimation step 213: The pharmacokinetic parameter estimation device 144 estimates a pharmacokinetic parameter on the basis of the data acquired at the data acquisition step 210 and the pharmacokinetic models built at the pharmacokinetic model building step 212.

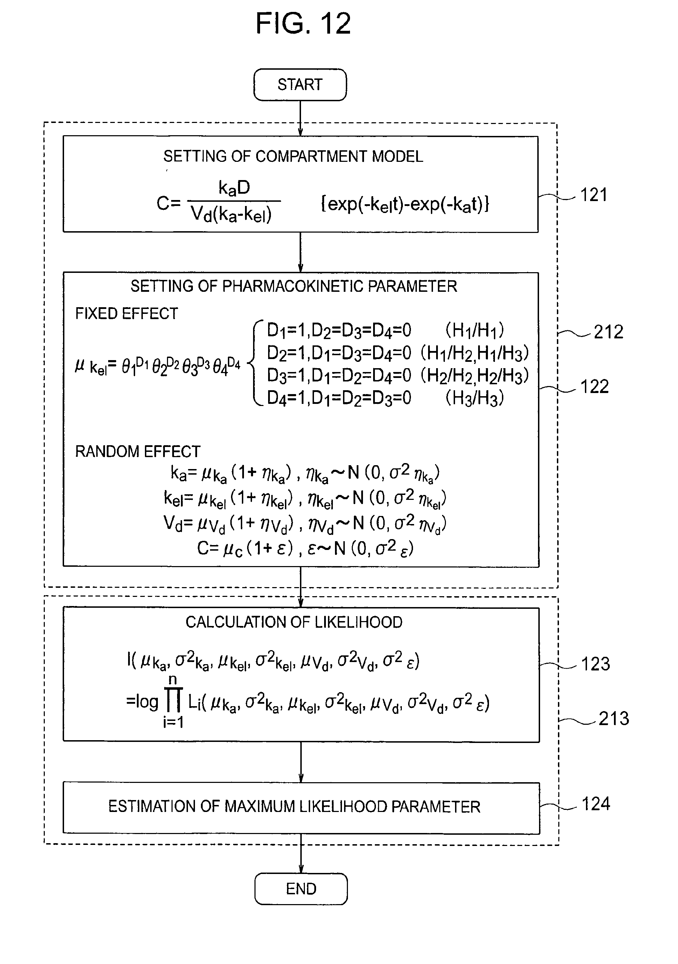

The details of the pharmacokinetic model building step 212 and the pharmacokinetic parameter estimation step 213 will be explained later using Fig. 12.

An information criterion calculation step 214: The optimum model selection device 145 calculates information criterion of the pharmacokinetic models on the basis of the pharmacokinetic models built at the pharmacokinetic model building step 212 and the pharmacokinetic parameter estimated at the pharmacokinetic parameter estimation step 213.

A model selection step 215: The optimum model selection device 145 judges whether or not the pharmacokinetic models built at the pharmacokinetic model building step 212 are optimum on the basis of the information criterion calculated at the information criterion calculation step 214, and the selection device 145 selects an optimum pharmacokinetic model.

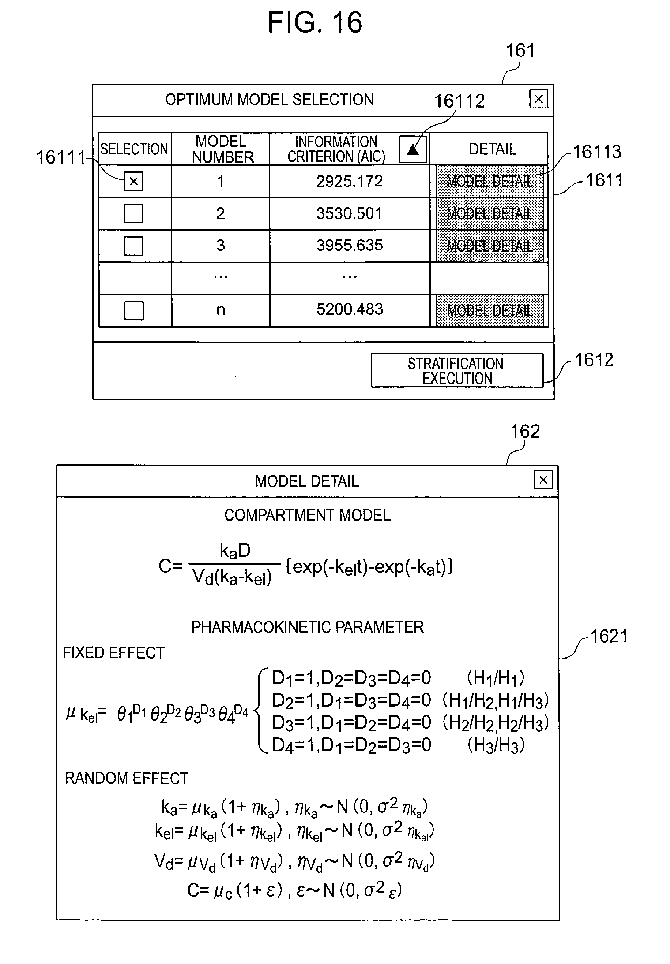

The details of the information criterion calculation step 214 and the model selection step 215 will be explained later using Fig. 16.

A drug-concentration time course curve creation step 216: The stratification device 146 creates a drug-concentration time course curve on the basis of the optimum pharmacokinetic model selected at the model selection step 215.

A stratification-condition satisfaction judgment step 217: The stratification device 146 judges whether or not the stratification condition 22 is satisfied based on the drug-concentration time course curve created at the drug-concentration time course curve creation step 216.

An analysis continuation judgment step 218: The stratification device 146 judges whether or not the pharmacokinetic analysis should be continued.

The details of the drug-concentration time course curve creation step 216, the stratification-condition satisfaction judgment step 217, and the analysis continuation judgment step 218 will be explained later.

An analysis result output step 219: The analysis result output device 147 outputs, as an analysis result 23, the data on the drug-concentration time course curve created at the drug-concentration time course curve creation step 216.

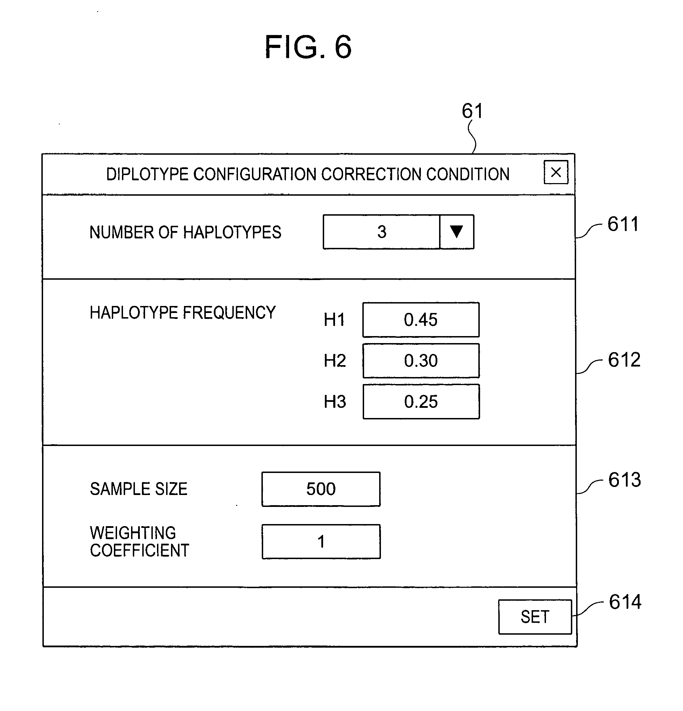

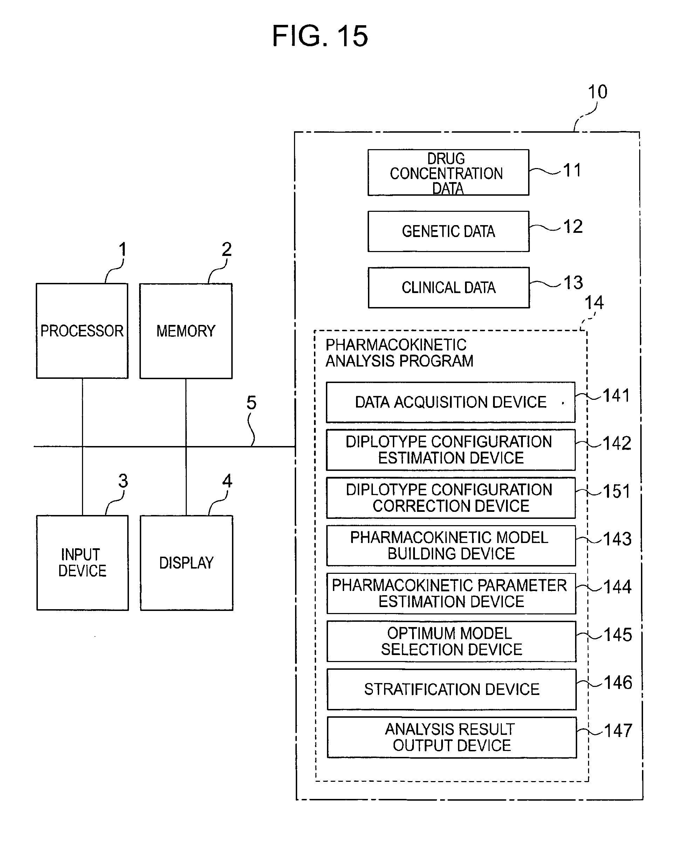

Fig. 3 is a diagram for illustrating an example of the embodiment of the pharmacokinetic analysis based on the pharmacokinetic analysis program 14 in a case of including a step of correcting the estimation result of the diplotype configurations using data created in a pseudo manner on the basis of given information on the haplotype frequency and the number of individuals (samples). Fig. 15 illustrates an example of the device configuration of the pharmacokinetic analysis device in the present embodiment. The device configuration of the pharmacokinetic analysis device in the present embodiment turns out to become a one where a diplotype configuration correction device 151 for correcting the estimation result of the diplotype configurations using data created in a pseudo manner on the basis of given information on the haplotype frequency and the number of individuals (samples) is added to the device configuration illustrated in Fig. 1. As illustrated in Fig. 3, the pharmacokinetic analysis based on the pharmacokinetic analysis program 14 in the present embodiment includes each step illustrated in Fig. 2 and a diplotype configuration correction step 310. At the diplotype configuration correction step 310, the diplotype configuration correction device 151 creates the data in a pseudo manner on the basis of haplotype frequency 31, sample size 32, and weighting coefficient 33 (indicating importance of documents or empirical knowledge) which are obtainable from documents, published database on the Web, empirical knowledge, and the like, and the correction device 151 corrects the estimation result of the diplotype configurations by using the data. The haplotype frequency 31, the sample size 32, and the weighting coefficient 33 are specified by the user via a setting screen which will be explained later using Fig. 6.

Fig. 4 is a diagram for illustrating an example of the screen of the pharmacokinetic analysis program 14 of the present invention. A pharmacokinetic analysis program screen 41 in the present embodiment includes a data input unit 411, a analysis condition setting button 412, a diplotype configuration correction condition setting button 413, and an analysis execution button 414.

In the data input unit 411, the drug concentration data 11, the genetic data 12, and the clinical data 13 are inputted which are needed for the data acquisition step 210 in Fig. 2 and Fig. 3. In the data input unit 411, e.g., a pass or the like to a file or database is inputted where the drug concentration data 11, the genetic data 12, and the clinical data 13 are stored.

Selecting and pressing the analysis condition setting button 412 makes it possible to set the population pharmacokinetic model building condition 21 and the stratification condition 22 needed for the pharmacokinetic model building step 212 in Fig. 2 and Fig. 3. The setting screen for the population pharmacokinetic model building condition 21 and the stratification condition 22 will be explained later using Fig. 5.

Selecting and pressing the diplotype configuration correction condition setting button 413 makes it possible to set the haplotype frequency 31, the sample size 32, and the weighting coefficient 33 needed for the diplotype configuration correction step 310 in Fig. 3. The setting screen for the haplotype frequency 31, the sample size 32, and the weighting coefficient 33 will be explained later using Fig. 6.

Selecting and pressing the analysis execution button 414 causes the pharmacokinetic analysis program 14 to start the pharmacokinetic analysis.

Fig. 5 is a diagram for illustrating an example of the setting screen of the population pharmacokinetic model building condition 21 and the stratification condition 22 needed for the pharmacokinetic model building step 212 in Fig. 2 and Fig. 3. An analysis condition setting screen 51 in the present embodiment includes a population pharmacokinetic model building condition input unit 511, a pharmacokinetic parameter setting unit 512, a stratification condition input unit 513, and a setting button 514.

In the population pharmacokinetic model building condition input unit 511, for example, like the screen in the present embodiment, a compartment model to be used for the pharmacokinetic analysis is selected by a pull-down button or the like. For example, like the present embodiment, when a 1-compartment model with oral administration is selected, pharmacokinetic parameters related with the selected 1-compartment model with oral administration are displayed on the pharmacokinetic parameter setting unit 512 together with check buttons and the like. In the present embodiment, absorption rate $K_a$, elimination rate $K_e$, distribution volume $V_d$, and in-blood drug concentration C are displayed as the pharmacokinetic parameters related with the 1-compartment model with oral administration. Of course, a pharmacokinetic parameter other than the pharmacokinetic parameters illustrated in Fig. 5, such as, e.g., clearance CL, may also be displayed. In the pharmacokinetic parameter setting unit 512, which pharmacokinetic parameter of the pharmacokinetic parameters should be expressed as a function of the diplotype configuration can be set in a manner of being classified into fixed effect and random effect. In the present embodiment, the fixed effect of the elimination rate $K_e$ is set as the function of the diplotype configuration. Also, in the pharmacokinetic parameter setting unit 512, an error model can be set together with the pharmacokinetic parameter. For example, like the present embodiment, the error model to be used for the pharmacokinetic analysis can be selected from among an absolute error model, a relative error model, an exponential error model, and a mixed error model. In the present embodiment, the relative error model is selected as the error model. In the stratification condition input unit 513, for example, like the screen in the present embodiment, a stratification condition for performing stratification of patients is selected by a pull-down button or the like. In the present embodiment, the stratification condition 22 is set assuming that the stratification of patients is performed when no overlapping exists in a 90-% confidence interval of the drug-concentration time course curve (i.e., no overlapping exists between lower-limit of the 90-% confidence interval of an upper-side drug-concentration time course curve and upper-limit of the 90-% confidence interval of a lower-side drug-concentration time course curve). As the stratification condition 22, a stratification condition other than the one indicated in the present embodiment may also be set, such as, e.g., the stratification is performed when an x or more difference exists in the drug concentrations, the stratification is performed when a difference between lower-limit of the 95-% confidence interval of the upper-side drug-concentration time course curve and upper-limit of the 95-% confidence interval of the lower-side drug-concentration time course curve is y or more, the stratification is performed regarding patients whose drug concentrations attain to a critical area and do not attain thereto. The population pharmacokinetic model building condition is set by the setting button 514.

Fig. 6 is a diagram for illustrating an example of the setting screen of the haplotype frequency 31, the sample size 32, and the weighting coefficient 33 needed for the diplotype configuration correction step 310 in Fig. 3. A diplotype configuration correction condition setting screen 61 in the present embodiment includes a haplotype number input unit 611, a haplotype frequency input unit 612, a sample-size/weighting-coefficient input unit 613, and a setting button 614.

In the haplotype number input unit 611, for example, like the screen in the present embodiment, the number of haplotypes existing in a genetic area becoming the analysis target is inputted by a pull-down button or the like. For example, like the present embodiment, if the number of the haplotypes is inputted as being 3, a screen for inputting frequencies of the respective haplotypes (which are denoted by $H_1$ to $H_3$ each, for example) is displayed on the haplotype frequency input unit 612. In the sample-size/weighting-coefficient input unit 613, the sample size 32 and the weighting coefficient 33 are inputted, and the diplotype configuration correction condition is set by the setting button 614. The user inputs the diplotype configuration correction condition on the basis of information obtainable from documents, published database on the Web, empirical knowledge, and the like.

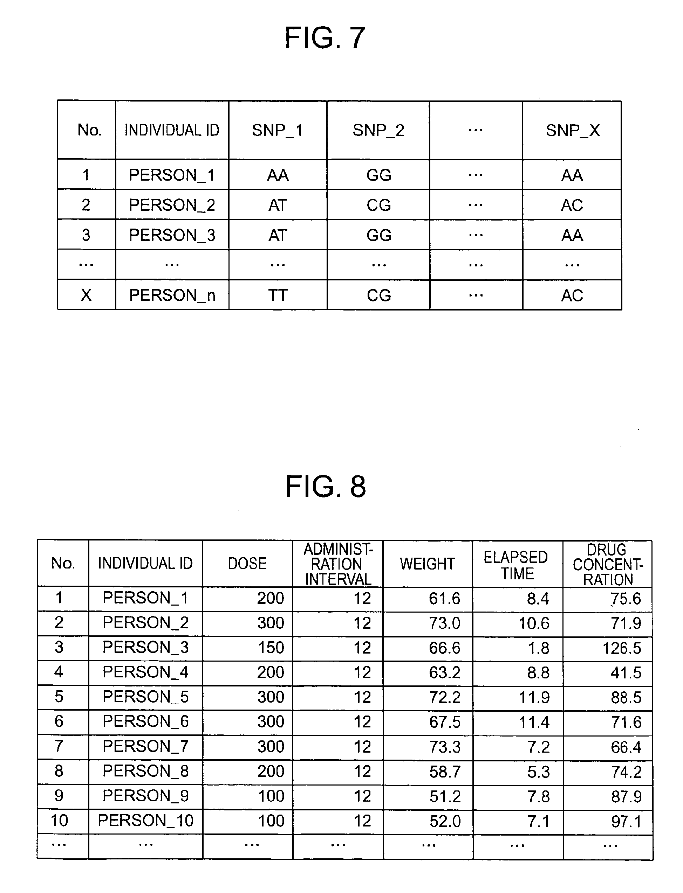

Fig. 7 is a diagram for illustrating an example of the data structure of the genetic data 12 of the present invention. Fig. 7 illustrates an example of the data structure of genetic polymorphism information on each individual basis. In the example in Fig. 7, genetic type information on the genetic polymorphisms which are X in number (all of which are assumed to be SNP polymorphisms in the present embodiment) are stored. For example, an individual PERSON_1 has two A alleles for a polymorphism SNP_1 (i.e., is homo of the A alleles for the polymorphism SNP_1), and has two G alleles for a polymorphism SNP_2 (i.e., is homo of the G alleles for the polymorphism SNP_2). Similarly, an individual PERSON_2 has one A allele and one T allele for the polymorphism SNP_1 (i.e., is hetero for the polymorphism SNP_1), and has one C allele and one G allele for the polymorphism SNP_2 (i.e., is hetero for the polymorphism SNP_2). Although Fig. 7 illustrates the example of the data on the SNP polymorphisms, data on another genetic polymorphism, such as ATRP polymorphism, may also be stored therein.

Fig. 8 is a diagram for illustrating an example of the data structure of the drug pharmacokinetic data 11 and the clinical data 13. Fig. 8 illustrates an example where the dose, administration interval, elapsed time, and in-blood drug concentration on each individual basis are stored as the drug pharmacokinetic data, and where the weight on each individual basis is stored as the clinical data. Although Fig. 7 illustrates the example where the drug pharmacokinetic data 11 and the clinical data 13 are stored into the same table, the drug pharmacokinetic data 11 and the clinical data 13 may also be stored in a separated manner.

Next, referring to Fig. 9, the explanation will be given below concerning an example of the embodiment of the diplotype configuration estimation step 211 in Fig. 2 and Fig. 3. In the present embodiment, the haplotype frequencies of a population and the diplotype configurations of individuals are estimated using the EM algorithm. Now, consider a sample group which includes n individuals. In this sample group, consider haplotypes in a plurality of marker locuses under linkage, and let frequencies of the haplotypes in the population be $F = (F_1, F_2, ..., F_M)$. Here, M is the total number of possible haplotypes. For example, if all of the marker locuses are SNP locuses, M = 2L holds, letting the number of the locuses be L. Observed data on the genetic type of each individual in the plurality of marker locuses under the linkage are represented by $G = (G_1, G_2, ..., G_n)$. In many cases, $G_i$ is incomplete data.

Accordingly, the diplotype configurations corresponding to $G_i$ are not determined into one configuration in many cases. In the case like this, posterior probability distributions (which will be referred to as "diplotype distributions") on possible diplotype configurations are defined. The diplotype configuration corresponding to $G_i$ for each individual i (i = 1, 2, ..., n) is represented by $D_{ij}$ (j = 1, 2, ..., $m_i$). Here, $m_i$ is the number of the possible diplotype configurations for $G_i$, and maximum value of $m_i$ is M. A storage area for storing the M possible haplotypes (which are denoted by $H_1, H_2, ..., H_M$ each) in the population and the frequencies F of the M possible haplotypes, and a storage area for storing the possible diplotype configuration $D_{ij}$ for each individual and the posterior probability distributions of the possible diplotype configuration $D_{ij}$ are ensured on the memory 2 on the basis of the genetic data 12 (which is equivalent to the above-described G) on the plurality of individuals acquired by the data acquisition device 141 in Fig. 1 and Fig. 15 at the data acquisition step 210 in Fig. 2 and Fig. 3. At the diplotype configuration estimation step 211 in Fig. 2 and Fig. 3, the diplotype configuration estimation device 142 in Fig. 1 and Fig. 15 estimates the diplotype configuration of each individual in association with the data stored on the memory 2 and in accordance with the following respective steps:

An initial value setting step 91: First, initial values F (0) of the haplotype frequencies are allocated to the M possible haplotypes (which are denoted by $H_1, H_2, ..., H_M$ each), then being stored into the storage area. The sum total of the haplotype frequencies is equal to 1. The initial value of each haplotype frequency is given at random such that the sum total of the haplotype frequencies becomes equal to 1. Otherwise, 1/M is equally given to each of the M possible haplotypes.

Next, with respect to 0, 1, 2, ..., F (t+1) is calculated from F(t) in accordance with the following diplotype distribution calculation step 92 to haplotype frequency update step 96:

The diplotype distribution calculation step 92: Each diplotype configuration $D_{ij}$ includes the two haplotypes $H_1$ and $H_m$, where $1 \leqq 1 \leqq M$ and $1 \leqq m \leqq M$. When the haplotype frequencies F(t) of the population are given, the probability with which $D_{ij}$ will be obtained is given by the following Expression (1):

(Expression 1)

$$\Pr(D_{ij}) = \begin{cases} F_l^{(t)2} & l = m \quad \text{if l = m} \\ 2F_l^{(t)} F_m^{(t)} & l \neq m \quad \text{if l } \neq \text{ m} \end{cases} \quad \text{---- (1)}$$

Consequently, the posterior probability $\Pr(D_{ij} | G_i)$ that the diplotype configuration of the individual i is $D_{ij}$ under the observed data $G_i$ on the genetic type is given by the following Expression (2) from Bayes' theorem:

(Expression 2)

$$\Pr(D_{ij}|G_i) = \frac{\Pr(D_{ij})\Pr(G_i|D_{ij})}{\sum_{k=1}^{m_i} \Pr(D_{ik})\Pr(G_i|D_{ik})} = \frac{\Pr(D_{ij})}{\sum_{k=1}^{m_i} \Pr(D_{ik})} \quad \text{---- (2)}$$

Calculating this Expression for all j (j = 1, 2, ..., $m_i$) determines the diplotype distribution of the individual i. This diplotype distribution is applied to all the individuals in the sample group, then being stored into the storage area.

A likelihood calculation step 93: The determination of the diplotype distribution of the individual i makes it possible to calculate the entire likelihood in the sample group. The entire likelihood can be expressed by the following Expression (3) by connecting likelihoods of all the diplotype configurations for each individual and further, by connecting likelihoods of all the individuals. A storage area for storing the likelihood is ensured on the memory 2, thereby storing the likelihood.

(Expression 3)

$$L(F^{(t)}) = \Pr(G|F^{(t)}) = \prod_{i=1}^{n} \sum_{j=1}^{m_i} \Pr(D_{ij}) \quad -\!\!-\ (3)$$

A haplotype-frequency expectation value calculation step 94: The determination of the diplotype distribution of the individual i makes it possible to calculate expectation values of the haplotype frequencies of the population from the diplotype distributions of all the individuals in the sample group. The expectation values of the haplotype frequencies of the population are given by the following Expression (4):

(Expression 4)

$$E[F_i^{(t)}] = \frac{1}{2n} \sum_{j=1}^{n} \sum_{k=1}^{m_i} \Pr(D_{jk}|G_j) N_{D_{j}ki} \quad -\!\!-\ (4)$$

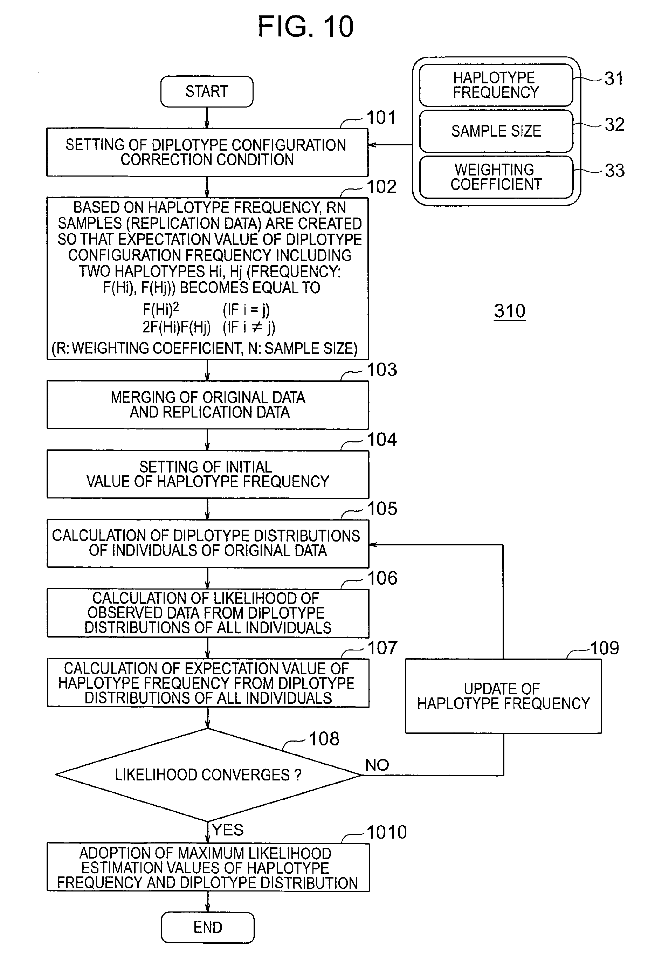

Here, $N_{Djki}$ is the number of $H_i$ (i.e., any one of 0, 1, and 2) included in the diplotype configuration $D_{jk}$.
A convergence judgment step 95: It is judged whether or not the value of the entire likelihood L(F) has been converged. If L(F(t+1))-L(F(t)) < β is satisfied, It is judged that the value has been converged, and the processing proceeds to the following diplotype distribution determination step 97: Meanwhile, if L(F(t+1))-L(F(t)) < β is not satisfied, the processing returns to the diplotype distribution calculation step 92 via the following haplotype frequency update step 96, then repeating the steps up to the convergence judgment step 95: Here, β is a threshold value. A sufficiently small value such as, e.g., $10^{-5}$ is set as the value of β.
The haplotype frequency update step 96: F is updated by setting F(t+1) = E[F(t)].
The diplotype distribution determination step 97: The value E[F] = F(EM) at the point-in-time when the entire likelihood has been converged is defined as maximum likelihood estimation value of the haplotype frequencies in the population. Moreover, the posterior probability Pr(DIG) at this time is defined as the diplotype distribution of the individual under the maximum likelihood estimation value of the haplotype frequencies in the population.
Next, referring to Fig. 10, the explanation will be given below concerning an example of the embodiment of the diplotype configuration correction step 310 in Fig. 3. In the present embodiment, the haplotype frequencies of a population and the diplotype configurations of individuals are estimated using the EM algorithm. In the present embodiment as well, consider the sample group which includes n individuals. Then, the haplotype frequencies F in the population, the observed data G on the genetic type in a plurality of marker locuses under linkage, and the diplotype configuration $D_{ij}$ (j = 1, 2, ..., $m_i$) corresponding to $G_i$ for each individual i (i = 1, 2, ..., n) are set exactly as described earlier. In the present embodiment as well, the storage area for storing the M possible haplotypes (which are denoted by $H_1$, $H_2$, ..., $H_M$ each) in the population and the frequencies F of the M possible haplotypes, and the storage area for storing the possible diplotype configuration $D_{ij}$ for each individual and the posterior probability distributions of the possible diplotype configuration $D_{ij}$ are ensured on the memory 2 on the basis of the genetic data 12 (which is equivalent to the above-described G) on the plurality of individuals acquired by the data acquisition device 141 in Fig. 15 at the data acquisition step 210 in Fig. 3.
A diplotype configuration correction condition acquisition step 101: The data on the haplotype frequency 31, the sample size 32, and the weighting coefficient 33 (indicating importance of documents or empirical knowledge) are acquired which are obtainable from documents, published database on the Web, empirical knowledge, and the like. The haplotype frequency 31, the sample size 32, and the weighting coefficient 33 are specified by the user via the setting screen explained earlier using Fig. 6.
A replication data creation step 102: Based on the data acquired at the diplotype configuration correction condition acquisition step 101, samples (individuals) which are RN in number are created in a pseudo manner so that expectation value of the diplotype configuration frequency including two haplotypes $H_i$ and $H_j$ (whose frequencies are set at $F(H_i)$ and $F(H_j)$ each) becomes equal to $F(H_i)^2$ if $i = j$, and $2F(H_i)F(H_j)$ if $i \neq j$. Here, R denotes the weighting coefficient 33, and N denotes the sample size 32.

In the present embodiment, the data on the individuals acquired in advance (observed actually) are referred to as "original data"; while the data on the individuals created in a pseudo manner at the replication data creation step 102 are referred to as "replication data".

A data merge step 103: The original data and the replication data are merged with each other. The genetic data 12 on the plurality of individuals acquired by the data acquisition device 141 and the replication data created in a pseudo manner at the replication data creation step 102 are added, thereby being formed into one piece of data.

A step 104 to a step 1010 are steps corresponding to the initial value setting step 91 to the diplotype distribution determination step 97 in Fig. 9. However, at the diplotype distribution calculation step 92 in Fig. 9, the diplotype distributions of all the individuals are calculated. In contrast thereto, the diplotype distribution calculation step 105 in Fig. 10 differs therefrom in the point that the diplotype distributions of the individuals of only the original data are calculated.

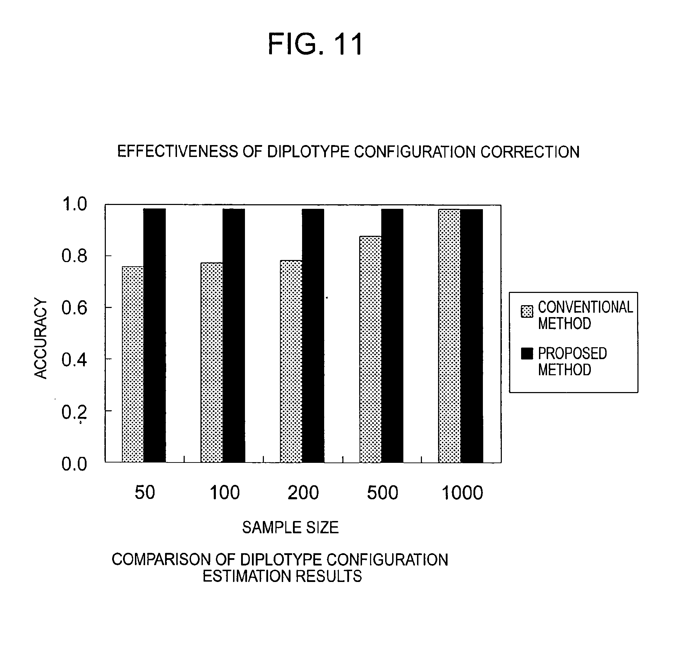

Fig. 11 is a diagram for illustrating effectiveness of the diplotype-configuration estimation result correction made by the diplotype configuration correction step 310. The longitudinal axis of the graph denotes accuracy of the diplotype configuration estimation result, and the transverse axis denotes the number of the samples (individuals). The accuracy is calculated by making a comparison between diplotype configuration data (correct-solution data) on individuals whose diplotype configurations are determined in advance, and diplotype configuration data created by dividing the correct-solution data into genetic type data in a plurality of marker locuses configuring the diplotype configuration, and re-estimating the diplotype configuration data from the genetic type data divided. The present invention (i.e., the proposed method) includes the diplotype configuration correction step 310. This makes it possible to obtain the high accuracy as compared with the conventional method even in the case where the sample size is small.

Next, referring to Fig. 12, the explanation will be given below concerning an example of the embodiment of the pharmacokinetic model building step 212 and the pharmacokinetic parameter estimation step 213 in Fig. 2 and Fig. 3. The embodiment illustrated in Fig. 12 includes a compartment model setting step 121, a pharmacokinetic parameter setting step 122, a likelihood calculation step 123, and a parameter estimation step 124. The compartment model setting step 121 and the pharmacokinetic parameter setting step 122 correspond to the pharmacokinetic model building step 212 in Fig. 2 and Fig. 3. The likelihood calculation step 123 and the parameter estimation step 124 correspond to the pharmacokinetic parameter estimation step 213 in Fig. 2 and Fig. 3.

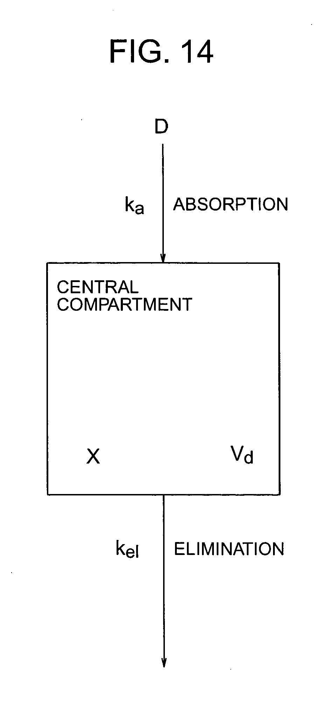

At the pharmacokinetic model building step 212, based on the population pharmacokinetic model building condition 21 and the stratification condition 22 specified by the user via the setting screen explained earlier using Fig. 5, the pharmacokinetic model building device 143 sets a compartment model at the compartment model setting step 121, and sets a pharmacokinetic parameter at the pharmacokinetic parameter setting step 122. In the present embodiment, the pharmacokinetic analysis is made using the 1-compartment model with oral administration as illustrated in Fig. 14. In the 1-compartment model with oral administration as illustrated in Fig. 14, the estimation value C of the in-blood drug concentration at the point-in-time when a time t has elapsed after the administration is represented by the following Expression (5):

(Expression 5)

$$C = \frac{k_a D}{V_d(k_a - k_{el})} \{\exp(-k_{el}t) - \exp(-k_a t)\} \quad \text{---} (5)$$

Here, D is the dose, $K_a$ is absorption rate, $K_{el}$ is elimination rate, and $V_d$ is distribution volume. The population pharmacokinetic analysis using the 1-compartment model with oral administration can be explained as follows: Now, assume that the in-blood drug concentration is measured for an individual i at $m_i$ points-in-time $t_{i1}, ..., t_{imi}$, and that the resultant measurement values are $x_{i1}, ..., x_{imi}$. Expressing all of pharmacokinetic parameters of the individual i as a vector $\theta_i$ in batch, the measurement values $x_{ij}$ of the in-blood drug concentration can be expressed as $x_{ij} = C(\theta_i, t_{ij}) + \varepsilon_{ij}$. This is because the measurement values $x_{ij}$ can be obtained by adding an error $\varepsilon_{ij}$, i.e., a probability variable, to the estimation value $C(\theta_i, t_{ij})$ based on the 1-compartment model with oral administration. In the case of the 1-compartment model with oral administration, the pharmacokinetic parameter vector of the individual i is $\theta_i = (k_a^{(i)}, k_{el}^{(i)}, V_d^{(i)})^T$. Accordingly, letting the dose of the individual i be $D_i$, the estimation value of the in-blood drug concentration at the point-in-time when a time $t_{ij}$ has elapsed after the administration is represented by the following Expression (6):

(Expression 6)

$$C(\theta_i, t_{ij}) = \frac{k_a^{(i)} D_i}{V_d^{(i)} \left(k_a^{(i)} - k_{el}^{(i)}\right)} \left\{\exp\left(-k_{el}^{(i)} t_{ij}\right) - \exp\left(-k_a^{(i)} t_{ij}\right)\right\} \quad — (6)$$

For simplicity, assuming that the error $\varepsilon_{ij}$ follows a normal distribution of average 0 and variance $\sigma_\varepsilon^2$, probability density function representing distribution of the measurement values $x_{i1}$, ..., $x_{imi}$ at the $m_i$ measurement points-in-time $t_{i1}$, ..., $t_{imi}$ can be represented by the following Expression (7):

(Expression 7)

$$f(x_{i1}, \cdots, x_{im_i} | k_a^{(i)}, k_{el}^{(i)}, V_d^{(i)}, \sigma_\varepsilon^2)$$

$$= \prod_{j=1}^{m_i} \frac{1}{\sqrt{2\pi\sigma_\varepsilon^2}} \exp\left\{-\frac{(x_{ij} - C(\theta_i, t_{ij}))^2}{2\sigma_\varepsilon^2}\right\}$$

$$= \prod_{j=1}^{m_i} \frac{1}{\sqrt{2\pi\sigma_\varepsilon^2}} \exp\left\{-\frac{\left\{x_{ij} - \frac{k_a^{(i)} D_i}{V_d^{(i)} \left(k_a^{(i)} - k_{el}^{(i)}\right)} \left\{\exp\left(-k_{el}^{(i)} t_{ij}\right) - \exp\left(-k_a^{(i)} t_{ij}\right)\right\}\right\}^2}{2\sigma_\varepsilon^2}\right\}$$

$$— (7)$$

For simplicity, the following normal distribution is assumed as a distribution for representing differences in the pharmacokinetic parameters between the individuals: $k_a^{(i)} \sim N(\mu_{ka}, \sigma_{ka}^2)$, $k_{el}^{(i)} \sim N(\mu_{kel}, \sigma_{kel}^2)$, $V_d^{(i)} \sim N(\mu_{Vd}, \sigma_{Vd}^2)$.

Although population parameters are unknown, likelihood function for the individual i is represented by the following Expression (8) under the situation that the measurement data $x_{i1}$, ..., $x_{imi}$ on the individual i are given: This representation is made possible by considering the above-described probability density function as a function of the unknown population parameters. What is referred to as "the unknown population parameters" here means the variance $\sigma_\varepsilon^2$ of the error, and $\mu_{ka}$, $\sigma_{ka}^2$, $\mu_{kel}$, $\sigma_{kel}^2$, $\mu_{Vd}$, and $\sigma_{Vd}^2$ which specify the distribution of the pharmacokinetic parameters in the population.

(Expression 8)

$$L_i\left(\mu_{k_a},\sigma^2_{k_a},\mu_{k_{el}},\sigma^2_{k_{el}},\mu_{V_d},\sigma^2_{V_d},\sigma^2_\varepsilon\right)$$

$$= \iiint \left[ \prod_{j=1}^{m_i} \frac{1}{\sqrt{2\pi\sigma_\varepsilon^2}} \exp\left\{ -\frac{\left\{ x_{ij} - \frac{k_a^{(i)} D_i}{V_d^{(i)}\left(k_a^{(i)} - k_{el}^{(i)}\right)} \left\{ \exp\left(-k_{el}^{(i)} t_{ij}\right) - \exp\left(-k_a^{(i)} t_{ij}\right) \right\} \right\}^2}{2\sigma_\varepsilon^2} \right\} \right.$$

$$\times \frac{1}{\sqrt{2\pi\sigma_{k_a}^2}} \exp\left\{ -\frac{\left(k_a^{(i)} - \mu_{k_a}\right)^2}{2\sigma_{k_a}^2} \right\}$$

$$\times \frac{1}{\sqrt{2\pi\sigma_{k_{el}}^2}} \exp\left\{ -\frac{\left(k_{el}^{(i)} - \mu_{k_{el}}\right)^2}{2\sigma_{k_{el}}^2} \right\}$$

$$\left. \times \frac{1}{\sqrt{2\pi\sigma_{V_d}^2}} \exp\left\{ -\frac{\left(V_d^{(i)} - \mu_{V_d}\right)^2}{2\sigma_{V_d}^2} \right\} \right] dk_a^{(i)} dk_{el}^{(i)} dV_d^{(i)} \qquad — (8)$$

If the measurement values at the $m_i$ points-in-time $t_{i1}$, ..., $t_{imi}$ for each individual i have been found to be $x_{i1}$, ..., $x_{imi}$, logarithmic likelihood function for all the data can be represented by the following Expression (9), letting the number of all the individuals be n:

(Expression 9)

$$l\left(\mu_{k_a},\sigma^2_{k_a},\mu_{k_{el}},\sigma^2_{k_{el}},\mu_{V_d},\sigma^2_{V_d},\sigma^2_\varepsilon\right)$$

$$= \log \prod_{i=1}^{n} L_i\left(\mu_{k_a},\sigma^2_{k_a},\mu_{k_{el}},\sigma^2_{k_{el}},\mu_{V_d},\sigma^2_{V_d},\sigma^2_\varepsilon\right) \qquad — (9)$$

In the population pharmacokinetic analysis, the population parameters $\mu_{ka}$, $\sigma_{ka}{}^2$, $\mu_{kel}$, $\sigma_{kel}{}^2$, $\mu_{vd}$, $\sigma_{vd}{}^2$, and $\sigma_\varepsilon{}^2$ which will maximize this logarithmic likelihood function are estimated. The analysis model to be used in the population pharmacokinetic analysis turns out to become a mixed effect model, which includes the fixed effect of the average values $\mu_{ka}$ and $\mu_{kel}$ of the population parameters and $\mu_{Vd}$, and the random effect of $\sigma_{ka}{}^2$, $\sigma_{kel}{}^2$, $\sigma_{vd}{}^2$, and $\sigma_\varepsilon{}^2$ whose average values are equal to zero.

At the pharmacokinetic model building step 212, the pharmacokinetic model is built by the following two steps:

The compartment model setting step 121: In accordance with the population pharmacokinetic model building condition 21 specified by the user via the setting screen explained earlier using Fig. 5, the 1-compartment model with oral administration is set based on the Expression (5).

The pharmacokinetic parameter setting step 122: In accordance with the population pharmacokinetic model building condition 21 specified by the user via the setting screen explained earlier using Fig. 5, the pharmacokinetic parameters are set. In the present embodiment, the population parameters $\mu_{ka}$, $\mu_{kel}$, and $\mu_{Vd}$ are expressed as functions of the diplotype configuration of each individual, and are considered to be the fixed effect of the mixed effect model, thereby configuring the population pharmacokinetic analysis model. For simplicity, in the present embodiment, it is assumed that the elimination of the drug is due to metabolism enzyme alone, and that the value of $\mu_{kel}$ depends on the diplotype configuration of each individual. The fixed effect and the random effect of the mixed effect model are set as the following Expression (10) and Expression (11), respectively:

(Expression 10)

$$\mu_{k_{el}} = \theta_1^{D_1} \theta_2^{D_2} \theta_3^{D_3} \theta_4^{D_4} \begin{cases} D_1 = 1, & D_2 = D_3 = D_4 = 0 & (H_1/H_1) \\ D_2 = 1, & D_1 = D_3 = D_4 = 0 & (H_1/H_2, H_1/H_3) \\ D_3 = 1, & D_1 = D_2 = D_4 = 0 & (H_2/H_2, H_2/H_3) \\ D_4 = 1, & D_1 = D_2 = D_3 = 0 & (H_3/H_3) \end{cases} \quad \text{---} (10)$$

(Expression 11)

$$k_a = \mu_{k_a}(1 + \eta_{k_a}), \quad \eta_{k_a} \sim N(0, \sigma_{\eta_{ka}}^2)$$

$$k_{el} = \mu_{k_{el}}(1 + \eta_{k_{el}}), \quad \eta_{k_{el}} \sim N(0, \sigma_{\eta_{k_{el}}}^2)$$

$$V_d = \mu_{V_d}(1 + \eta_{V_d}), \quad \eta_{V_d} \sim N(0, \sigma_{\eta_{V_d}}^2) \qquad \text{---} (11)$$

$$C = \mu_C(1 + \varepsilon), \quad \varepsilon \sim N(0, \sigma_\varepsilon^2)$$

Here, $H_i$ (i = 1, 2, 3) denote the haplotypes, and $H_i/H_j$ (i = 1, 2, 3, j = 1, 2, 3) denote the diplotype configurations including the haplotypes $H_i$ and $H_j$. In the present embodiment, the three haplotypes are considered, then setting the fixed effect of $\mu_{kel}$ on each diplotype-configuration basis.

At the pharmacokinetic parameter estimation step 213, the pharmacokinetic parameter estimation device 144 calculates the likelihood at the likelihood calculation step 123, and estimates the pharmacokinetic parameters at the parameter estimation step 124. Hereinafter, the explanation will be given below regarding the likelihood calculation step 123 and the parameter estimation step 124.

The likelihood calculation step 123: A storage area for storing the likelihood of each individual i to be calculated by the Expression (8) is ensured on the memory 2. This calculation is made on the basis of the drug concentration data 11 and the clinical data 13 (which are equivalent to the above-described D, $(t_{i1}, ..., t_{imi})$, and $(x_{i1}, ..., x_{imi})$) on the plurality of individuals acquired by the data acquisition device 141 in Fig. 1 and Fig. 15 at the data acquisition step 210 in Fig. 2 and Fig. 3, and the diplotype configuration data (which are equivalent to the above-described $H_i$ and $H_j$) estimated by the diplotype configuration estimation device 142 in Fig. 1 and Fig. 15 at the diplotype configuration estimation step 211 in Fig. 2 and Fig. 3. Next, the likelihood of each individual i is calculated by the Expression (8), then being stored into the storage area. Moreover, a storage area for storing the logarithmic likelihood for all the data to be calculated by the Expression (9) is ensured on the memory 2. Furthermore, the logarithmic likelihood for all the data is calculated based on the Expression (9), then being stored into the storage area.

The parameter estimation step 124: The parameters which will maximize the logarithmic likelihood calculated at the likelihood calculation step 123 are estimated. Then, the parameters estimated are employed as the pharmacokinetic parameters in the population. The methods such as maximum likelihood method and EM algorithm are used for the parameter estimation.

Next, the explanation will be given below concerning an example of the embodiment of the information criterion calculation step 214 in Fig. 2 and Fig. 3. Although, in the present embodiment, Akaike Information Criterion (: AIC) is used as the information criterion, some other information criterion may also be used. Using maximum logarithmic likelihood of a model and free parameter number of the model, AIC can be calculated by the following Expression: -2 * (maximum logarithmic likelihood of a model) + 2 * (free parameter number of the model). At the information criterion calculation step 214, the optimum model selection device 145 calculates AIC on the basis of the pharmacokinetic models built at the pharmacokinetic model building step 212 and the pharmacokinetic parameters estimated at the pharmacokinetic parameter estimation step 213. Using the above-described Expression, AIC is calculated from the maximum logarithmic likelihood and the pharmacokinetic parameter number calculated at the likelihood calculation step 123 and the parameter estimation step 124 in Fig. 12.

Fig. 16 is a diagram for illustrating an example of the screen for selecting an optimum pharmacokinetic model by the optimum model selection device 145 at the model selection step 215. An optimum model selection screen 161 illustrated at the upper portion in Fig. 16 includes an information criterion display unit 1611 and a stratification execution button 1612.

The information criterion display unit 1611 displays calculation results of AIC of the respective models built by the pharmacokinetic model building device 143 at the pharmacokinetic model building step 212. Using a sort button 16112 makes it possible to sort the calculation results in an ascendant or descendent order.

Pressing a model detail button 16113 displays a model detail display screen 162 illustrated at the lower portion in Fig. 16. This screen makes it possible to know details of a model selected. The model detail display screen 162 includes a model detail display unit 1621.

The user selects the optimum model on the basis of the calculation results of AIC, then checking a selection model check box 16111 and pressing the stratification execution button 1612. This starts the stratification by the stratification device 146. In the present embodiment, the example has been indicated where the user selects the optimum model using the optimum model selection screen 161. Instead of performing the interactive processing with the user, however, the optimum model selection device 145 may automatically select the model which will make AIC the minimum, and start the stratification by the stratification device 146.

Next, the explanation will be given below concerning the drug-concentration time course curve creation step 216, the stratification-condition satisfaction judgment step 217, and the analysis continuation judgment step 218 in Fig. 2 and Fig. 3.

At the drug-concentration time course curve creation step 216, the drug-concentration time course curve is created on the basis of the optimum pharmacokinetic model selected by the optimum model selection device 145 at the model selection step 215 in Fig. 2 and Fig. 3. Moreover, at the stratification-condition satisfaction judgment step 217, it is judged whether or not the drug-concentration time course curve created satisfies the stratification condition 22 specified by the user via the setting screen explained earlier using Fig. 5.

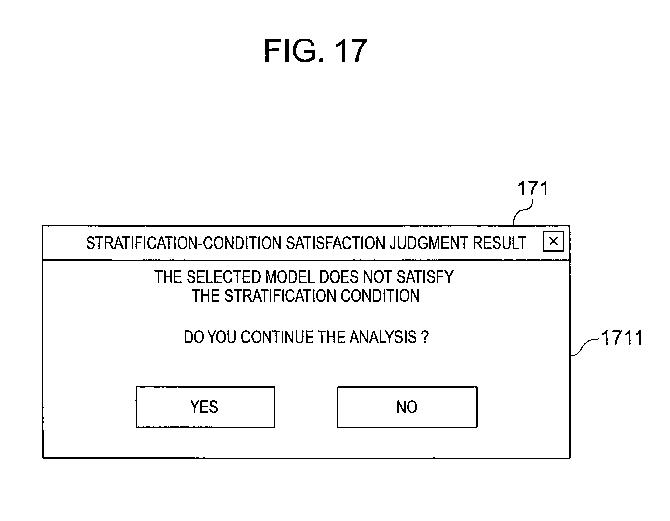

At the stratification-condition satisfaction judgment step 217, the stratification device 146 judges whether or not the stratification condition 22 is satisfied based on the drug-concentration time course curve created at the drug-concentration time course curve creation step 216. As a result of this, if the stratification condition 22 is satisfied, the processing proceeds to the analysis result output step 219, where the analysis result output device 147 outputs the analysis result 23. Also, at the stratification-condition satisfaction judgment step 217, the stratification device 146 judges whether or not the stratification condition 22 is satisfied based on the drug-concentration time course curve created at the drug-concentration time course curve creation step 216. As a result of this, if the stratification condition 22 is not satisfied, as the analysis continuation judgment step 218, a stratification-condition satisfaction judgment result screen 171 as is illustrated in, e.g., Fig. 17 is displayed. The stratification-condition satisfaction judgment result screen 171 includes a stratification-condition satisfaction judgment result display unit 1711. Pressing "Yes" button returns the processing to the pharmacokinetic model building step 212, thereby continuing the pharmacokinetic analysis. Pressing "No" button terminates the pharmacokinetic analysis.

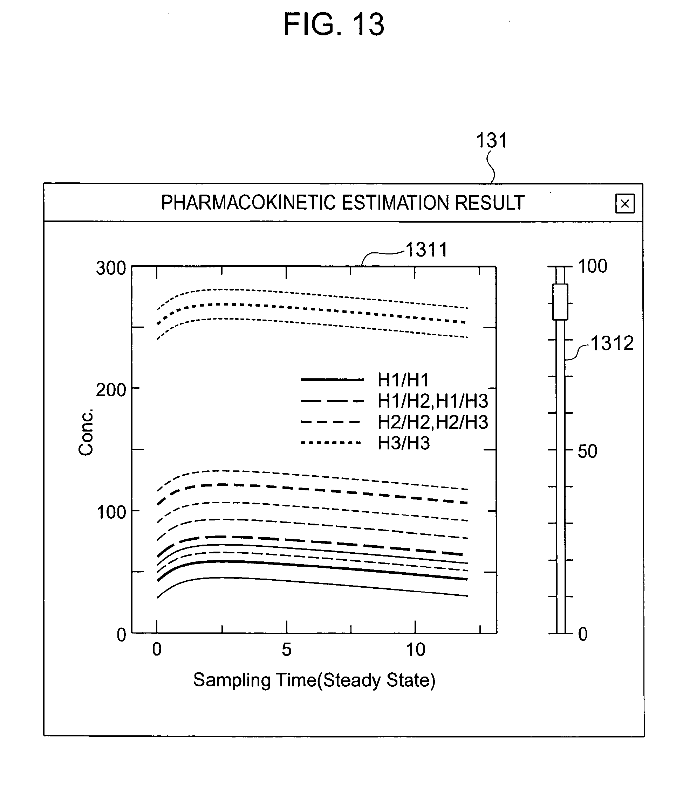

Fig. 13 is a diagram for illustrating an example of the screen of the analysis result 23 outputted by the analysis result output step 219 in Fig. 2 and Fig. 3. A pharmacokinetic estimation result output screen 131 in the present embodiment includes a drug-concentration time course curve display unit 1311 and a confidence interval setting bar 1312.

The drug-concentration time course curve display unit 1311 displays the data on the drug-concentration time course curve created at the drug-concentration time course curve creation step 216 in Fig. 2 and Fig. 3. In the example of the screen in the present embodiment, graphs of drug-concentration time course curves for, e.g., four groups are displayed. Here, the four groups are an individual having the diplotype configuration of $H_1/H_1$, an individual having the diplotype configuration of $H_1/H_2$ or $H_1/H_3$, an individual having the diplotype configuration of $H_2/H_2$ or $H_2/H_3$, and an individual having the diplotype configuration of $H_3/H_3$. The longitudinal axis of the graph denotes in-blood drug concentration, and the transverse axis denotes elapsed time in stationary state. Each drug-concentration time course curve is displayed with the confidence interval.

The confidence interval setting bar 1312 sets the confidence interval of each drug-concentration time course curve displayed on the drug-concentration time course curve display unit 1311. The upper-limit of the confidence interval is 100 %, and the lower-limit thereof is 0 %. In the example of the screen in the present embodiment, the confidence interval setting bar 1312 is set at, e.g., 90 %. Accordingly, each drug-concentration time course curve (bold line) is displayed on the drug-concentration time course curve display unit 1311 together with the upper-limit and lower-limit (narrow lines) of the 90-% confidence interval.

The user is permitted to freely set the confidence interval of each drug-concentration time course curve displayed as the analysis result. This feature allows the user to obtain information for performing the stratification of patients depending on a purpose.

It should be further understood by those skilled in the art that although the foregoing description has been made on embodiments of the invention, the invention is not limited thereto and various changes and modifications may be made without departing from the spirit of the invention and the scope of the appended claims.

**Claims**

1.  A pharmacokinetic analysis system, comprising:

    input means (3),
    a database for storing drug concentration data (11), genetic data (12), and clinical data (13),
    data acquisition means (141) for acquiring said drug concentration data (11), said genetic data (12), and said clinical data (13) from said database, and on said basis of information inputted by said input means (3),
    diplotype configuration estimation means (142) for estimating diplotype configurations of a plurality of individuals on said basis of said genetic data (12) on said plurality of individuals acquired by said data acquisition means (141),
    pharmacokinetic model building means (143) for building pharmacokinetic models on said basis of said diplotype configurations of said individuals estimated by said diplotype configuration estimation means (142),
    pharmacokinetic parameter estimation means (144) for estimating pharmacokinetic parameters on said basis of said data acquired by said data acquisition means (141) and said pharmacokinetic models built by said pharmacokinetic model building means (143),
    optimum model selection means (145) for calculating information criterion of said pharmacokinetic models on said basis of said pharmacokinetic models built by said pharmacokinetic model building means (143) and said pharmacokinetic parameters estimated by said pharmacokinetic parameter estimation means (144), judging whether or not said pharmacokinetic models built by said pharmacokinetic model building means (143) are optimum on said basis of said information criterion calculated, and selecting an optimum pharmacokinetic model,
    stratification means (146) for creating a drug-concentration time course curve on said basis of said optimum pharmacokinetic model selected by said optimum model selection means (145), and performing stratification of said drug-concentration time course curve, and
    output means (147) for outputting, as an analysis result, said data on said drug-concentration time course curve created by said stratification means (146).

2.  The pharmacokinetic analysis system according to Claim 1, further comprising:

    diplotype configuration correction means (151) for correcting said diplotype configurations estimated by said diplotype configuration estimation means (142), wherein
    said diplotype configuration correction means (151) creates replication data on said basis of haplotype frequency (31), sample size (32), and weighting coefficient (33), and corrects said diplotype configuration estimation result on said basis of said replication data created, said haplotype frequency (31), said sample size (32), and said weighting coefficient (33) being diplotype configuration correction conditions set by said input means (3).

3.  The pharmacokinetic analysis system according to Claim 2, wherein
    said diplotype configuration estimation result is corrected by merging said replication data and original data with each other, and calculating diplotype distributions of individuals of said original data, said original data being data on individuals acquired in advance.

4.  The pharmacokinetic analysis system according to Claim 1, wherein said diplotype configuration estimation means (142)
    extracts number of haplotypes possible in a population and frequencies of said haplotypes, and diplotype configuration possible for each individual and posterior probability of said diplotype configuration on said basis of said genetic data (12) on said plurality of individuals acquired,
    allocates initial values of said haplotype frequencies to said haplotypes,
    calculates diplotype distributions of all of said individuals,
    calculates likelihood of said entire samples and expectation value of said haplotype frequencies using said calculated diplotype distributions of all of said individuals,
    judges whether or not value of said likelihood of said entire samples has been converged, and
    estimates said diplotype configuration of each individual as maximum likelihood estimation value of said haplotype frequencies in said population at a point-in-time when said value had been converged.

**5.** The pharmacokinetic analysis system according to Claim 1, wherein

said input means (3) includes means (51) for setting a population pharmacokinetic model building condition (21) and a stratification condition (22),

said pharmacokinetic model building means (143) building said pharmacokinetic models on said basis of said population pharmacokinetic model building condition (21) and stratification condition (22) set by said means (51).

**6.** The pharmacokinetic analysis system according to Claim 5, further comprising:

means (41) for setting said pharmacokinetic parameters, wherein

said pharmacokinetic model building means (143) builds said pharmacokinetic models on said basis of said pharmacokinetic parameters set by said means (41) .

**7.** The pharmacokinetic analysis system according to Claim 1, wherein said pharmacokinetic parameter estimation means (144)

calculates likelihood of each individual on said basis of said drug concentration data (11) and said clinical data (13) on said plurality of individuals acquired by said data acquisition means (141), and said diplotype configuration data estimated by said diplotype configuration estimation means (142),

calculates logarithmic likelihood for all of said data from said calculated likelihood of each individual,

estimates parameters which maximize said calculated logarithmic likelihood, and

employs said estimated parameters as said pharmacokinetic parameters in said population.

**8.** The pharmacokinetic analysis system according to Claim 1, wherein

said output means (147) outputs said data on said drug-concentration time course curve together with a confidence interval.

**9.** A pharmacokinetic analysis method, comprising the steps of:

a data acquisition step (210) of acquiring drug concentration data (11), genetic data (12), and clinical data (13) from a database for storing said drug concentration data (11), said genetic data (12), and said clinical data (13), and on said basis of information inputted by input means (3),

a diplotype configuration estimation step (211) of estimating diplotype configurations of a plurality of individuals on said basis of said acquired genetic data (12) on said plurality of individuals,

a pharmacokinetic model building step (212) of building pharmacokinetic models on said basis of said estimated diplotype configurations,

a pharmacokinetic parameter estimation step (213) of estimating pharmacokinetic parameters on said basis of said acquired data and said built pharmacokinetic models,

an optimum model selection step (214, 215) of calculating information criterion of said pharmacokinetic models on said basis of said built pharmacokinetic models and said estimated pharmacokinetic parameters, judging whether or not said pharmacokinetic models are optimum on said basis of said calculated information criterion, and selecting an optimum pharmacokinetic model,

a stratification step (216, 217) of creating a drug-concentration time course curve on said basis of said selected optimum pharmacokinetic model, and performing stratification of said drug-concentration time course curve, and

an output step (219) of outputting said data on said drug-concentration time course curve as an analysis result.

# FIG. 1

10

DRUG CONCENTRATION DATA ~11

GENETIC DATA ~12

CLINICAL DATA ~13

14

1
PROCESSOR

2
MEMORY

5

3
INPUT DEVICE

4
DISPLAY

PHARMACOKINETIC ANALYSIS PROGRAM

DATA ACQUISITION DEVICE ~141

DIPLOTYPE CONFIGURATION ESTIMATION DEVICE ~142

PHARMACOKINETIC MODEL BUILDING DEVICE ~143

PHARMACOKINETIC PARAMETER ESTIMATION DEVICE ~144

OPTIMUM MODEL SELECTION DEVICE ~145

STRATIFICATION DEVICE ~146

ANALYSIS RESULT OUTPUT DEVICE ~147

EP 1 843 269 A1

# FIG. 2

START

DATA ACQUISITION — 210

ESTIMATION OF DIPLOTYPE CONFIGURATION — 211

PHARMACOKINETIC MODEL BUILDING — 212

ESTIMATION OF PHARMACOKINETIC PARAMETER — 213

CALCULATION OF INFORMATION CRITERION OF MODEL — 214

MODEL IS OPTIMUM ? — 215
NO / YES

DRAWING OF DRUG-CONCENTRATION TIME COURSE CURVE — 216

RESULT SATISFIES STRATIFICATION CONDITION ? — 217
NO / YES

USER CONTINUES ANALYSIS ? — 218
YES / NO

OUTPUT OF RESULT — 219

END

DRUG CONCENTRATION DATA — 11
GENETIC DATA — 12
CLINICAL DATA — 13

POPULATION PHARMACOKINETIC MODEL BUILDING CONDITION — 21
STRATIFICATION CONDITION — 22

RESULT — 23

# FIG. 3

START

→ 210

DATA ACQUISITION

DRUG CONCENTRATION DATA — 11

GENETIC DATA — 12

CLINICAL DATA — 13

→ 211

ESTIMATION OF DIPLOTYPE CONFIGURATION

→ 310

CORRECTION OF DIPLOTYPE CONFIGURATION USING EXTERNAL INFORMATION

HAPLOTYPE FREQUENCY — 31

SAMPLE SIZE — 32

WEIGHTING COEFFICIENT — 33

→ 212

PHARMACOKINETIC MODEL BUILDING

POPULATION PHARMACOKINETIC MODEL BUILDING CONDITION — 21

STRATIFICATION CONDITION — 22

→ 213

ESTIMATION OF PHARMACOKINETIC PARAMETER

→ 214

CALCULATION OF INFORMATION AMOUNT CRITERION OF MODEL

→ 215

MODEL IS OPTIMUM ?    NO

YES → 216

DRAWING OF DRUG-CONCENTRATION TIME COURSE CURVE

→ 217

RESULT SATISFIES STRATIFICATION CONDITION ?    NO

YES

→ 218

USER CONTINUES ANALYSIS ?    YES

NO

→ 219

OUTPUT OF RESULT → RESULT — 23

NO

END

# FIG. 4

41

PHARMACOKINETIC ANALYSIS PROGRAM | × |

DRUG
CONCENTRATION DATA | | REFERENCE | 411

GENETIC DATA | | REFERENCE |

CLINICAL DATA | | REFERENCE |

CONDITION SETTING | 412

DIPLOTYPE CORRECTION | 413

START | 414

# FIG. 5

51

| ANALYSIS CONDITION | ⊠ |
|---|---|

POPULATION PHARMACOKINETIC MODEL BUILDING CONDITION

511

COMPARTMENT MODEL | 1-COMPARTMENT, ORAL ADMINISTRATION ▼ |

DIPLOTYPE FIXED EFFECT ☐ ka ⊠ ke ☐ Vd ☐ C

DIPLOTYPE RANDOM EFFECT ☐ ka ☐ ke ☐ Vd ☐ C

512

ERROR MODEL ☐ABSOLUTE ⊠RELATIVE☐EXPONENTIAL☐MIXED

STRATIFICATION CONDITION

513

| NO OVERLAPPING IN 90-% CONFIDENCE INTERVAL ▼ |

| SET | 514

# FIG. 6

61

| DIPLOTYPE CONFIGURATION CORRECTION CONDITION | ☒ |

| | |
| --- | --- |
| NUMBER OF HAPLOTYPES | 3 ▼ |

611

| HAPLOTYPE FREQUENCY | H1 | 0.45 |
| | H2 | 0.30 |
| | H3 | 0.25 |

612

| SAMPLE SIZE | 500 |
| WEIGHTING COEFFICIENT | 1 |

613

| | SET |

614

# FIG. 7

| No. | INDIVIDUAL ID | SNP_1 | SNP_2 | ... | SNP_X |
|---|---|---|---|---|---|
| 1 | PERSON_1 | AA | GG | ... | AA |
| 2 | PERSON_2 | AT | CG | ... | AC |
| 3 | PERSON_3 | AT | GG | ... | AA |
| ... | ... | ... | ... | ... | ... |
| X | PERSON_n | TT | CG | ... | AC |

# FIG. 8

| No. | INDIVIDUAL ID | DOSE | ADMINIST-RATION INTERVAL | WEIGHT | ELAPSED TIME | DRUG CONCENT-RATION |
|---|---|---|---|---|---|---|
| 1 | PERSON_1 | 200 | 12 | 61.6 | 8.4 | 75.6 |
| 2 | PERSON_2 | 300 | 12 | 73.0 | 10.6 | 71.9 |
| 3 | PERSON_3 | 150 | 12 | 66.6 | 1.8 | 126.5 |
| 4 | PERSON_4 | 200 | 12 | 63.2 | 8.8 | 41.5 |
| 5 | PERSON_5 | 300 | 12 | 72.2 | 11.9 | 88.5 |
| 6 | PERSON_6 | 300 | 12 | 67.5 | 11.4 | 71.6 |
| 7 | PERSON_7 | 300 | 12 | 73.3 | 7.2 | 66.4 |
| 8 | PERSON_8 | 200 | 12 | 58.7 | 5.3 | 74.2 |
| 9 | PERSON_9 | 100 | 12 | 51.2 | 7.8 | 87.9 |
| 10 | PERSON_10 | 100 | 12 | 52.0 | 7.1 | 97.1 |
| ... | ... | ... | ... | ... | ... | ... |

# FIG. 9

211

```
        ┌─────────┐
        │  START  │
        └─────────┘
             │
             ▼
┌─────────────────────────────┐  ─ 91
│  SETTING OF INITIAL VALUE OF │
│     HAPLOTYPE FREQUENCY      │
└─────────────────────────────┘
             │
             ▼
┌─────────────────────────────┐  ─ 92
│    CALCULATION OF DIPLOTYPE  │ ◄───────────────┐
│  DISTRIBUTIONS OF ALL INDIVIDUALS │             │
└─────────────────────────────┘                  │
             │                                    │
             ▼                                    │
┌─────────────────────────────┐  ─ 93            │
│    CALCULATION OF LIKELIHOOD OF │               │
│      OBSERVED DATA FROM DIPLOTYPE │             │
│   DISTRIBUTIONS OF ALL INDIVIDUALS │            │
└─────────────────────────────┘                  │
             │                                    │
             ▼                                    │
┌─────────────────────────────┐  ─ 94   ┌──────────────────────┐ ─ 96
│  CALCULATION OF EXPECTATION VALUE OF │ │      UPDATE OF        │
│ HAPLOTYPE FREQUENCY FROM DIPLOTYPE │   │  HAPLOTYPE FREQUENCY  │
│  DISTRIBUTIONS OF ALL INDIVIDUALS │    └──────────────────────┘
└─────────────────────────────┘                  ▲
             │                                    │
             ▼                                    │
        ◇─────────────◇  ─ 95                     │
       ╱               ╲       NO                 │
      ╱  LIKELIHOOD     ╲──────────────────────────┘
      ╲  CONVERGES ?    ╱
       ╲               ╱
        ◇─────────────◇
             │ YES
             ▼
┌─────────────────────────────┐  ─ 97
│ ADOPTION OF MAXIMUM LIKELIHOOD │
│  ESTIMATION VALUES OF HAPLOTYPE │
│ FREQUENCY AND DIPLOTYPE DISTRIBUTION │
└─────────────────────────────┘
             │
             ▼
        ┌─────────┐
        │   END   │
        └─────────┘
```

# FIG. 10

START

↓

**101** SETTING OF DIPLOTYPE CONFIGURATION CORRECTION CONDITION

↓

**102** BASED ON HAPLOTYPE FREQUENCY, RN SAMPLES (REPLICATION DATA) ARE CREATED SO THAT EXPECTATION VALUE OF DIPLOTYPE CONFIGURATION FREQUENCY INCLUDING TWO HAPLOTYPES Hi, Hj (FREQUENCY: F(Hi), F(Hj)) BECOMES EQUAL TO

$$F(Hi)^2 \quad (IF\ i = j)$$
$$2F(Hi)F(Hj) \quad (IF\ i \neq j)$$

(R: WEIGHTING COEFFICIENT, N: SAMPLE SIZE)

↓

**103** MERGING OF ORIGINAL DATA AND REPLICATION DATA

↓

**104** SETTING OF INITIAL VALUE OF HAPLOTYPE FREQUENCY

↓

**105** CALCULATION OF DIPLOTYPE DISTRIBUTIONS OF INDIVIDUALS OF ORIGINAL DATA

↓

**106** CALCULATION OF LIKELIHOOD OF OBSERVED DATA FROM DIPLOTYPE DISTRIBUTIONS OF ALL INDIVIDUALS

↓

**107** CALCULATION OF EXPECTATION VALUE OF HAPLOTYPE FREQUENCY FROM DIPLOTYPE DISTRIBUTIONS OF ALL INDIVIDUALS

↓

**108** LIKELIHOOD CONVERGES ? — NO → **109** UPDATE OF HAPLOTYPE FREQUENCY

↓ YES

**1010** ADOPTION OF MAXIMUM LIKELIHOOD ESTIMATION VALUES OF HAPLOTYPE FREQUENCY AND DIPLOTYPE DISTRIBUTION

↓

END

**31** HAPLOTYPE FREQUENCY

**32** SAMPLE SIZE

**33** WEIGHTING COEFFICIENT

310

# FIG. 11

EFFECTIVENESS OF DIPLOTYPE CONFIGURATION CORRECTION

COMPARISON OF DIPLOTYPE CONFIGURATION
ESTIMATION RESULTS

# FIG. 12

START

SETTING OF COMPARTMENT MODEL

$$C = \frac{k_a D}{V_d(k_a - k_{el})} \{exp(-k_{el}t) - exp(-k_a t)\}$$

— 121

SETTING OF PHARMACOKINETIC PARAMETER

FIXED EFFECT

$$\mu_{k_{el}} = \theta_1^{D_1} \theta_2^{D_2} \theta_3^{D_3} \theta_4^{D_4} \begin{cases} D_1=1, D_2=D_3=D_4=0 & (H_1/H_1) \\ D_2=1, D_1=D_3=D_4=0 & (H_1/H_2, H_1/H_3) \\ D_3=1, D_1=D_2=D_4=0 & (H_2/H_2, H_2/H_3) \\ D_4=1, D_1=D_2=D_3=0 & (H_3/H_3) \end{cases}$$

— 212

— 122

RANDOM EFFECT

$$k_a = \mu_{k_a}(1 + \eta_{k_a}), \quad \eta_{k_a} \sim N(0, \sigma^2_{\eta_{k_a}})$$
$$k_{el} = \mu_{k_{el}}(1 + \eta_{k_{el}}), \quad \eta_{k_{el}} \sim N(0, \sigma^2_{\eta_{k_{el}}})$$
$$V_d = \mu_{V_d}(1 + \eta_{V_d}), \quad \eta_{V_d} \sim N(0, \sigma^2_{\eta_{V_d}})$$
$$C = \mu_c(1 + \varepsilon), \quad \varepsilon \sim N(0, \sigma^2_\varepsilon)$$

CALCULATION OF LIKELIHOOD

$$l(\mu_{k_a}, \sigma^2_{k_a}, \mu_{k_{el}}, \sigma^2_{k_{el}}, \mu_{V_d}, \sigma^2_{V_d}, \sigma^2_\varepsilon)$$

$$= \log \prod_{i=1}^{n} L_i(\mu_{k_a}, \sigma^2_{k_a}, \mu_{k_{el}}, \sigma^2_{k_{el}}, \mu_{V_d}, \sigma^2_{V_d}, \sigma^2_\varepsilon)$$

— 123

— 213

ESTIMATION OF MAXIMUM LIKELIHOOD PARAMETER

— 124

END

# FIG. 13

# FIG. 14

D

$k_a$   ABSORPTION

CENTRAL
COMPARTMENT

X                    $V_d$

$k_{el}$   ELIMINATION

# FIG. 15

# FIG. 16

**OPTIMUM MODEL SELECTION** — 161, 16112 — [×]

| SELECTION | MODEL NUMBER | INFORMATION CRITERION (AIC) ▲ | DETAIL |
|---|---|---|---|
| [×] | 1 | 2925.172 | MODEL DETAIL |
| ☐ | 2 | 3530.501 | MODEL DETAIL |
| ☐ | 3 | 3955.635 | MODEL DETAIL |
| | ... | ... | |
| ☐ | n | 5200.483 | MODEL DETAIL |

16111 — 16113 — 1611

STRATIFICATION EXECUTION — 1612

---

**MODEL DETAIL** — 162 — [×]

## COMPARTMENT MODEL

$$C = \frac{k_a D}{V_d(k_a - k_{el})} \left[ \exp(-k_{el}t) - \exp(-k_a t) \right]$$

## PHARMACOKINETIC PARAMETER

**FIXED EFFECT**

$$\mu_{k_{el}} = \theta_1^{D_1} \theta_2^{D_2} \theta_3^{D_3} \theta_4^{D_4} \begin{cases} D_1=1, D_2=D_3=D_4=0 & (H_1/H_1) \\ D_2=1, D_1=D_3=D_4=0 & (H_1/H_2, H_1/H_3) \\ D_3=1, D_1=D_2=D_4=0 & (H_2/H_2, H_2/H_3) \\ D_4=1, D_1=D_2=D_3=0 & (H_3/H_3) \end{cases}$$

**RANDOM EFFECT**

$$k_a = \mu_{k_a}(1 + \eta_{k_a}), \quad \eta_{k_a} \sim N(0, \sigma^2_{\eta_{k_a}})$$

$$k_{el} = \mu_{k_{el}}(1 + \eta_{k_{el}}), \quad \eta_{k_{el}} \sim N(0, \sigma^2_{\eta_{k_{el}}})$$

$$V_d = \mu_{V_d}(1 + \eta_{V_d}), \quad \eta_{V_d} \sim N(0, \sigma^2_{\eta_{V_d}})$$

$$C = \mu_c(1 + \varepsilon), \quad \varepsilon \sim N(0, \sigma^2_\varepsilon)$$

— 1621

# FIG. 17

171

| STRATIFICATION-CONDITION SATISFACTION JUDGMENT RESULT | ☒ |
| --- | --- |

THE SELECTED MODEL DOES NOT SATISFY
THE STRATIFICATION CONDITION

DO YOU CONTINUE THE ANALYSIS ?

1711

| YES | NO |
| --- | --- |

EP 1 843 269 A1

**European Patent Office**

**EUROPEAN SEARCH REPORT**

Application Number

EP 07 00 5630

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| P,X | KUNG-HAO LIANG ET AL: "An algorithm for model construction and its applications to pharmacogenomic studies" JOURNAL OF HUMAN GENETICS, SPRINGER-VERLAG, TO, vol. 51, no. 9, 10 August 2006 (2006-08-10), pages 751-759, XP019385695 ISSN: 1435-232X * the whole document * | 1-9 | INV. G06F19/00 |
| X | SULTATOS LESTER G ET AL: "Incorporation of the genetic control of alcohol dehydrogenase into a physiologically based pharmacokinetic model for ethanol in humans." TOXICOLOGICAL SCIENCES : AN OFFICIAL JOURNAL OF THE SOCIETY OF TOXICOLOGY MAR 2004, vol. 78, no. 1, 12 January 2004 (2004-01-12), pages 20-31, XP002435190 ISSN: 1096-6080 * abstract; figures 1,2,5-11; tables 2,3 * * page 20, left-hand column, paragraph 1 - right-hand column, paragraph 1 * * page 21, left-hand column, paragraph 2 - page 26, right-hand column, paragraph 2 * | 1-9 | TECHNICAL FIELDS SEARCHED (IPC)   G06F |

-/--

The present search report has been drawn up for all claims

| Place of search | Date of completion of the search | Examiner |
|---|---|---|
| The Hague | 29 May 2007 | Swarén, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

32

**European Patent Office**

**EUROPEAN SEARCH REPORT**

Application Number

EP 07 00 5630

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KITAMURA Y ET AL: "DETERMINATION OF PROBABILITY OF DIPLOTYPE OF DIPLOTYPE CONFIGURATION (DIPLOTYPE DISTRIBUTION) FOR EACH SUBJECT FROM GENOTYPIC DATA USING THE EM ALGORITHM" ANNALS OF HUMAN GENETICS, CAMBRIDGE UNIVERSITY PRESS, LONDON, GB, vol. 66, no. PART 3, May 2002 (2002-05), pages 183-193, XP009032701 ISSN: 0003-4800 * abstract * * page 183, right-hand column, last paragraph - page 184, left-hand column, paragraph 1 * * page 186, left-hand column, paragraph 1 - right-hand column, paragraph 1 * ----- | 1-9 | |

TECHNICAL FIELDS SEARCHED (IPC)

The present search report has been drawn up for all claims

| Place of search | Date of completion of the search | Examiner |
|---|---|---|
| The Hague | 29 May 2007 | Swarén, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 1 843 269 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **EXCOFFIER L ; SLATKIN M.** Maximum-likelihood estimation of molecular haplotype frequencies in a diploid population. *Mol Biol Evol,* 1995, vol. 12, 921-927

- **STEPHENS M et al.** A new statistical method for haplotype reconstruction from population data. *Am J Hum Genet,* 2001, vol. 68, 978-989